(19) Europäisches Patentamt
European Patent Office
Office européen des brevets

(11) Veröffentlichungsnummer: **0 359 160 A2**

# (12) EUROPÄISCHE PATENTANMELDUNG

(21) Anmeldenummer: **89116727.2**

(22) Anmeldetag: **09.09.89**

(51) Int. Cl.5: **C07C 303/40 , C07C 311/29 , C07C 311/38**

(30) Priorität: **15.09.88 DE 3831382**

(43) Veröffentlichungstag der Anmeldung: **21.03.90 Patentblatt 90/12**

(84) Benannte Vertragsstaaten: **CH DE FR GB IT LI NL**

(71) Anmelder: **HOECHST AKTIENGESELLSCHAFT**
**Postfach 80 03 20**
**D-6230 Frankfurt am Main 80(DE)**

(72) Erfinder: **Tronich, Wolfgang, Dr.**
**Adolf-Guckes-Weg 5**
**D-6239 Eppstein/Taunus(DE)**
Erfinder: **Forstinger, Klaus, Dr.**
**Bergstrasse 38**
**D-6092 Kelsterbach(DE)**
Erfinder: **Hertel, Hasso, Dr.**
**Brunnenweg 10**
**D-6052 Mühlheim/Main(DE)**

## (54) Verfahren zur Herstellung von 2-Alkoxy-benzolsulfonamid-Verbindungen.

(57) 2-Alkoxy-benzolsulfonamid-Verbindungen entsprechend der allgemeinen Formel

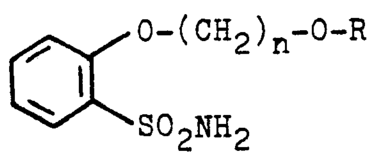

in welcher R für eine Alkylgruppe von 1 bis 4 C-Atomen und n für die Zahl 2, 3 oder 4 steht, werden hergestellt, indem man das Diazoniumsalz einer 5-Amino-2-alkoxy-benzolsulfonamid-Verbindung der allgemeinen Formel

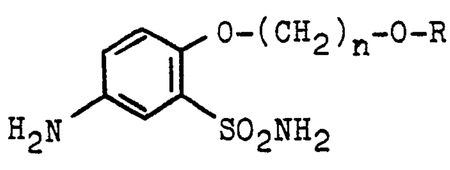

mit R und n der angegebenen Bedeutung mit einer reduzierend wirkenden Verbindung umsetzt. Reduzierend wirkende Verbindungen sind insbesondere Hypophosphorige Säure, gegebenenfalls in Anwesenheit von Kupfer oder einer kupferhaltigen Verbindung, niedere Alkanole in Gegenwart von Kupfer oder einer kupferhaltigen Verbindung oder ein Gemisch solcher Agenzien, desweiteren Ameisensäure und saure wäßrige Formaldehydlösungen.

EP 0 359 160 A2

## Verfahren zur Herstellung von 2-Alkoxy-benzolsulfonamid-Verbindungen

Die Erfindung liegt auf dem technischen Gebiet der Zwischenprodukte.

2-Alkoxy-benzolsulfonamid-Verbindungen und deren Synthesen sind aus den US-Patentschriften Nrs. 4 419 121 und 4 479 821 sowie aus den europäischen Patentanmeldungs-Veröffentlichungen Nrs. 0 044 807A und 0 237 480A bekannt. Die drei erstgenannten Schriften beschreiben teils mehrstufige Herstellungsverfahren, die für die industrielle Anwendung wenig geeignet sind, weil entweder zur Synthese der Zwischenstufen Sandmeyer-Reaktionen mit unzureichender Selektivität angewandt werden müssen oder Produktgemische entstehen, die aufwendige Trennmethoden erfordern, oder aber das Verfahren mit dem Nachteil behaftet ist, daß während der einzelnen Verfahrensschritte pyrophores elementares Natrium oder halogenierte Kohlenwasserstoffe als Alkylierungsmittel oder Lösemittel eingesetzt werden. Daß die bekannten Verfahrensweisen für die technische Anwendung wenig geeignet sind, wird bereits in der anfangs ebenfalls zitierten europäischen Patentanmeldungs-Veröffentlichung Nr. 0 237 480A dargelegt.

Aber auch das letztgenannte, gegenüber diesem Stand der Technik verbesserte Verfahren zur Herstellung von 2-Alkoxy-benzosulfonamiden ist für die großtechnische Anwendung nicht von dem gewünschten Vorteil, da auch hier zur Synthese der 2-Alkoxy-benzolsulfonamid-Verbindung eine mehrstufige Verfahrensweise erforderlich ist, wobei ebenfalls halogenierte Kohlenwasserstoffe als Lösemittel eingesetzt werden.

Mit der vorliegenden Erfindung wurde nun ein neuer Syntheseweg zur Herstellung von 2-Alkoxy-benzolsulfonamid-Verbindungen entsprechend der allgemeinen Formel (1)

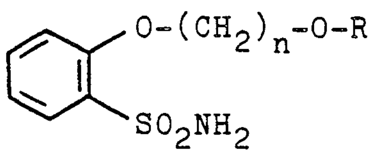

(1)

in welcher R eine Alkylgruppe von 1 bis 4 C-Atomen ist und n für die Zahl 2, 3 oder 4 steht, gefunden, mit welchem eine selektive Synthese dieser Verbindungen möglich ist und technisch schwer handhabbare oder ökologisch problematische Reagentien vermieden werden.

Entgegen der in J.Chem.Soc. 1958, 2903, beschriebenen Lehre, daß sich die Aminogruppe eines aminosubstituierten o-Hydroxy-benzolsulfonamids nicht durch Diazotierung und anschließende Reduktion mittels Hypophosphit eliminieren läßt, wurde nun überraschenderweise gefunden, daß man eine 2-Alkoxy-benzolsulfonamid-Verbindung der allgemeinen Formel (1) auf einfache Weise herstellen kann, wenn man das Diazoniumsalz einer 5-Amino-2-alkoxy-benzolsulfonamid-Verbindung der allgemeinen Formel (2)

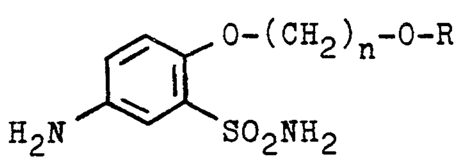

(2)

mit n und R der oben angegebenen Bedeutung mit einer reduzierend wirkenden Verbindung, wie beispielsweise einem Hypophosphit, umsetzt.

Reduzierend wirkende Verbindungen, die erfindungsgemäß im vorliegenden Verfahren eingesetzt werden können, sind beispielsweise saure wäßrige Formaldehydlösungen, Ameisensäure, bevorzugt in wäßriger Lösung, sowie deren Alkalimetallsalze, deren Ester mit aliphatischen Alkoholen, wie niederen Alkanolen, oder deren Amide, Hypophosphorige Säure, Alkanole von 1 bis 4 C-Atomen in Gegenwart von Kupfer oder Kupferverbindungen, wie Oxide und Salze des Kupfers, beispielsweise Kupfersulfat, Kupferchlorid, Kupferacetat, Kupferoxid, basisches Kupferoxid und basische Kupfersalze, wie basisches Kupferacetat, vorzugsweise in wäßrigem Medium, desweiteren Gemische aus Hypophosphoriger Säure mit $C_1$-$C_4$-Alkanolen, vorzugsweise in wäßrigem Medium, gegebenenfalls in Gegenwart von Kupfer oder kupferhaltigen Verbindungen, wie den obengenannten Verbindungen.

Bevorzugt wird erfindungsgemäß als reduzierend wirkendes Agenz Hypophosphorige Säure (als wäßrige Lösung) oder ein Gemisch dieser Hypophosphorigen Säure mit einem Alkanol von 1 bis 4 C-Atomen, jeweils gegebenenfalls in Gegenwart von Kupfer oder einer kupferhaltigen Verbindung, oder ein Alkanol von

1 bis 4 C-Atomen in Gegenwart von Kupfer oder einer kupferhaltigen Verbindung eingesetzt.

Die reduktive Abspaltung der Diazoniumgruppe ist aber auch durch katalytische Hydrierung mittels Wasserstoff in Gegenwart von Metallen der 8. Nebengruppe des Periodensystems, wie beispielsweise Palladium, möglich, jedoch sind die anderen erfindungsgemäßen Verfahrensvarianten dieser aus technischer Sicht vorzuziehen.

Das Diazoniumsalz der Ausgangsverbindung der Formel (2) erhält man in üblicher Weise durch Diazotierung der Aminoverbindung der Formel (2), beispielsweise in saurer wäßriger Lösung mittels Natriumnitrit bei einer Temperatur zwischen -10° C und +10° C. Als Säuren können beispielsweise Schwefelsäure, Salzsäure und Ameisensäure verwendet werden. In der Regel erfolgt die Umsetzung mit dem reduzierend wirkenden Agenz in der wäßrigen Syntheselösung des Diazoniumsalzes selbst; man kann jedoch auch von einer Diazoniumsalzlösung ausgehen, die durch Diazotierung der Aminoverbindung der Formel (2) in Ameisensäure erhalten wurde. Es ist auch möglich, von einem als stabiles Salz isolierten Diazoniumsalz der Aminoverbindung (2) auszugehen, wegen des hierfür zusätzlichen Verfahrensschrittes jedoch wenig zweckmäßig.

Die Reduktion mittels Hypophosphoriger Säure erfolgt in wäßriger Lösung, beispielsweise in der handelsüblichen wäßrigen Hypophosphorigen Säure. Pro Mol Diazoniumsalz werden mindestens 1 Mol Hypophosphoriger Säure eingesetzt; vorteilhaft verwendet man einen größeren Überschuß an Hypophosphoriger Säure, wie beispielsweise einen Überschuß zwischen 2 und 20 Mol, vorzugsweise zwischen 5 und 10 Mol, pro Mol Diazoverbindung. Die Umsetzung kann bei einer Temperatur zwischen -20° C und +30° C durchgeführt werden; vorzugsweise arbeitet man bei einer Temperatur zwischen -10° C und +10° C.

Eine bevorzugte Verfahrensvariante der Umsetzung des Diazoniumsalzes mit Hypophopphoriger Säure ist die Durchführung dieser Reaktion in Gegenwart von Kupfer oder einer kupferhaltigen Verbindung, wie beispielsweise den obengenannten Verbindungen.

Die erfindungsgemäße Umsetzung der Diazoniumverbindung mit einem Alkanol von 1 bis 4 C-Atomen, wie Methanol und Ethanol, in Gegenwart von Kupfer oder einer kupferhaltigen Verbindung, wie den obengenannten Verbindungen, führt man bei einer Temperatur zwischen 0° C und 85° C, vorzugsweise zwischen 50 und 70° C, durch. Zweckmäßig setzt man hierbei mindestens 5 Gew.-Teile des Alkanols pro 1 Gew.-Teil Ausgangsaminoverbindung (2), im allgemeinen zwischen 8 und 80 Gew.-Teilen, bevorzugt zwischen 8 und 30 Gewichtsteilen Alkanol pro 1 Gewichtsteil der Aminoverbindung (2), ein.

Zweckmäßig arbeitet man in der Weise, daß man die wäßrige, saure Diazoniumsalzlösung des Amins der Formel (2) langsam zu dem auf eine Temperatur zwischen 40 und 80° C, bevorzugt zwischen 50 und 70° C, erwärmten Alkanol, das zudem das Kupfer oder die kupferhaltige Verbindung enthält, hinzugibt. Die Umsetzung ist dann, wie auch bei der Reduktion mittels Hypophosphoriger Säure, in verhältnismäßig kurzer Zeit beendet.

Insbesondere bevorzugt ist die erfindungsgemäße Umsetzung mittels einem Gemisch aus Hypophosphoriger Säure und einem Alkanol von 1 bis 4 C-Atomen, wobei hierbei bevorzugt in Gegenwart von Kupfer oder einer kupferhaltigen Verbindung, wie einer der oben angegebenen Verbindungen, gearbeitet wird. Die Umsetzung erfolgt bei einer Temperatur zwischen -20° C und +30° C, bevorzugt zwischen -10° C und +10° C. Das Mengenverhältnis zwischen Hypophosphoriger Säure, dem Alkanol und der Ausgangsaminoverbindung (2) kann in weiten Mengen variieren, sofern das reduzierend wirkende Agenz in der mindestens äquimolaren Menge eingesetzt wird. Durch die Verwendung des Alkanols wird es möglich, die Menge an Hypophosphoriger Säure zu verringern; so kann man bereits vorteilhaft mit einem 2- bis 5-fach molaren Überschuß an Hypophosphoriger Säure pro Mol Diazoniumverbindung arbeiten. Die jeweils optimal einzusetzenden Mengen an Hypophospßoriger Säure und Alkanol können je nach Art der Ausgangs-Aminoverbindung (2) und der Art des Alkanols durch einfache Vorversuche ermittelt werden.

Eine weitere erfindungsgemäße Verfahrensvariante ist die Umsetzung mittels Ameisensäure als reduzierend wirkendem Agenz, so beispielsweise durch Vermischen der wäßrigen Diazoniumsalzlösung mit Ameisensäure oder mit Natriumformiat, wobei auch hier in Gegenwart von Kupfer oder einer kupferhaltigen Verbindung gearbeitet werden kann. Die Umsetzung verläuft bei Temperaturen zwischen 40 und 95° C.

Hierbei kann man vorteilhaft so verfahren, daß man von einer Lösung des Amins der Formel (2) in Ameisensäure ausgeht und dieses mittels einer wäßrigen Natriumnitritlösung in üblicher Weise diazotiert; auf diese Weise erhält man sogleich den eigentlichen Reaktionsansatz. Die Umsetzung des Diazoniumsalzes des Amins der Formel (2) mit der Ameisensäure zum Endprodukt der Formel (1) ist in verhältnismäßig kurzer Zeit abgeschlossen.

Das Kupfer oder die kupferhaltige Verbindung wird bevorzugt in katalytischen Mengen eingesetzt, so beispielsweise in einer Menge von 0,05 bis 3 Gew.-%, bevorzugt zwischen 0,3 und 2 Gew.-%, bezogen auf das Ausgangsamin der Formel (2).

Die erfindungsgemäß hergestellten 2-Alkoxy-benzolsulfonamide der Formel (1) lassen sich auf üblichem Wege aus den Syntheseansätzen isolieren. Aus wäßriger Lösung scheiden sie sich im allgemeinen bereits im Verlauf der Umsetzung in fester Form ab und können anschließend durch Filtration isoliert werden. Verwendet man ein Alkanol, so ist es zweckmäßig, die erhaltene Syntheselösung zunächst durch Abdestillation eines Teils des Alkanols zu konzentrieren; nach Abkühlen der Lösung kristallisiert die Verbindung (1) daraus aus.

Die Ausgangsverbindungen der Formel (2) sind technisch leicht verfügbar. Sie sind beispielsweise in der japanischen patentanmeldungs Veröffentlichung Sho-55-54350, referiert in Chem. Abstr. 93, 115919u (1980), beschrieben.

Die nachstehenden Beispiele dienen zur Erläuterung der Erfindung. Die Teile sind Gewichtsteile und die Prozentangaben stellen Gewichtsprozente dar, sofern nicht anders vermerkt. Gewichtsteile stehen im Verhältnis zu Volumenteilen wie Kilogramm zu Liter.

**Beispiel 1**

24,6 Teile 5-Amino-2-(2′-methoxy-ethoxy)-benzolsulfonamid werden in 95 Teilen einer 13,4%igen wäßrigen Salzsäure suspendiert. Nach Zusatz von 26 Teilen einer 30%igen wäßrigen Salzsäure gibt man bei einer Temperatur zwischen 0 °C und -5 °C langsam innerhalb von [illegible] Minuten 23,5 Teile einer 30%igen wäßrigen Natriumnitritlösung. Man rührt noch 15 Minuten nach und versetzt sodann die Diazoniumsalzlösung bei einer Temperatur zwischen 0 und 5 °C langsam innerhalb von 30 Minuten mit 132 Teilen einer 50%igen wäßrigen Hypophosphorigen Säure. Man rührt den Ansatz noch 20 Stunden nach und filtriert sodann das ausgefallene Produkt ab, wäscht es mit Wasser neutral und trocknet es.

Es werden 17,2 Teile 2(2′-Methoxy-ethoxy)-benzolsulfonamid mit einem Schmelzpunkt von 109-112 °C erhalten.

**Beispiel 2**

123 Teile 5-Amino-2′(2′-methoxy-ethoxy)-benzolsulfonamid werden in 412,5 Teilen einer 18,5%igen wäßrigen Salzsäure suspendiert. Die Diazotierung erfolgt bei 0 bis -5 °C durch Zugabe von 117,5 Teilen einer 30%igen wäßrigen Natriumnitritlösung innerhalb von 30 Minuten. Der Ansatz wird noch 15 Minuten nachgerührt und die Diazoniumsalzlösung sodann langsam innerhalb von 3 Stunden bei 0 °C bis -5 °C in 660 Teile einer 50%igen wäßrigen Hypophosphorigen Säure, die 1 Teil kristallines Kupfersulfat enthält, gegeben. Man rührt noch 2 Stunden bei dieser Temperatur nach, filtriert das ausgefallene Produkt ab, wäscht es neutral und trocknet es.

Es werden 87 Teile 2-(2′-Methoxy-ethoxy)-benzolsulfonamid mit einem Schmelzpunkt von 109-111 °C erhalten.

**Beispiel 3**

12,3 Teile 5-Amino-2-(2′-methoxy-ethoxy)-benzolsulfonamid werden in 39,2 Teilen einer 25%igen wäßrigen Schwefelsäure suspendiert. Man kühlt anschließend auf 0 °C bis -5 °C ab und diazotiert durch Zugabe von 11,5 Teilen einer 30%igen wäßrigen Natriumnitritlösung in üblicher Weise. Sodann gibt man die Diazoniumsalzlösung innerhalb von 15 Minuten langsam in 150 Volumenteile Methanol von 60 bis 65 °C, dem 0,1 Teile Kupferschliff zugefügt sind. Man hält die Temperatur während der Zugabe und rührt anschließend noch 15 Minuten weiter. Danach destilliert man 110 Volumenteile Methanol ab, kühlt den Ansatz auf 20 °C, wobei das Produkt teilweise auskristallisiert, und vervollständigt die Kristallisation durch Zugabe von 50 Teilen Eiswasser. Man filtriert ab, wäscht mit Wasser neutral und trocknet den Rückstand.

Man erhält 9,0 Teile 2-(2′-Methoxy-ethoxy)-benzolsulfonamid mit einem Schmelzpunkt von 110-111 °C.

**Beispiel 4**

123 Teile 5-Amino-2-(2′-methoxy-ethoxy)-benzolsulfonamid werden in 367,5 Teilen einer 15,3 %igen wäßrigen Salzsäure suspendiert; die Diazotierung erfolgt in üblicher Weise mittels 114,5 Teilen einer 30%igen wäßrigen Natriumnitritlösung. Die Diazoniumsalzlösung wird sodann innerhalb von 1 Stunde

langsam bei einer Temperatur zwischen -5° C und 0° C in ein Gemisch aus 500 Volumenteilen Methanol, 132,5 Teilen einer 50%igen wäßrigen Hypophosphorigen Säure und 0,5 Teilen Kupferschliff gegeben. Man rührt noch 30 Minuten nach und verdünnt den Syntheseansatz mit 500 Teilen Eiswasser. Nach weiterem 30-minütigem Rühren filtriert man das kristallin ausgefallene Produkt ab, wäscht es mit Wasser neutral und trocknet es.

Man erhält 90,1 Teile 2-(2'-Methoxy-ethoxy)-benolsulfonamid mit einem Schmelzpunkt von 110-111° C.

## Ansprüche

1. Verfahren zur Herstellung einer 2-Alkoxy-benzolsulfonamid-Verbindung entsprechend der allgemeinen Formel (1)

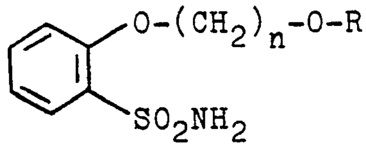

(1)

in welcher R für eine Alkylgruppe von 1 bis 4 C-Atomen und n für die Zahl 2, 3 oder 4 steht, dadurch gekennzeichnet, daß man das Diazoniumsalz einer 5-Amino-2-alkoxy-benzolsulfonamid-Verbindung der allgemeinen Formel (2)

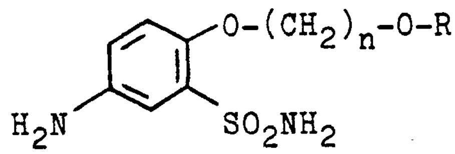

(2)

mit R und n der oben angegebenen Bedeutung mit einer reduzierend wirkenden Verbindung umsetzt.

2. Verfahren nach Anspruch 1, dadurch gekennzeichnet, daß das reduzierend wirkende Agenz Hypophosphorigen Säure ist.

3. Verfahren nach Anspruch 1, dadurch gekennzeichnet, daß das reduzierende wirkende Agenz Hypophosphorigen Säure in Gegenwart von Kupfer oder einer kupferhaltigen Verbindung ist.

4. Verfahren nach Anspruch 1, dadurch gekennzeichnet, daß das reduzierend wirkende Agenz ein Alkanol von 1 bis 4 C-Atomen in Gegenwart von Kupfer oder einer kupferhaltigen Verbindung ist.

5. Verfahren nach Anspruch 1, dadurch gekennzeichnet, daß das reduzierende Agenz ein Gemisch aus Hypophosphorigen Säure und einem Alkanol von 1 bis 4 C-Atomen ist.

6. Verfahren nach Anspruch 1, dadurch gkennzeichnet, daß das reduzierend wirkende Agenz ein Gemisch aus Hypophosphoriger Säure, einem Alkanol von 1 bis 4 C-Atomen und Kupfer oder einer kupferhaltigen Verbindung ist.

7. Verfahren nach Anspruch 1, dadurch gekennzeichnet, daß das reduzierend wirkende Agenz Ameisensäure oder ein Salz der Ameisensäure, wie Natriumformiat, ist.

8. Verfahren nach Anspruch 7, dadurch gekennzeichnet, daß die Umsetzung in Gegenwart von Kupfer oder einer kupferhaltigen Verbindung durchgeführt wird.